Europäisches Patentamt

**European Patent Office** (11) Publication number: **0 196 515**

Office européen des brevets **A1**

(12) **EUROPEAN PATENT APPLICATION**

(21) Application number: **86103411.4**

(22) Date of filing: **13.03.86**

(51) Int. Cl.⁴: **A 61 L 2/08**
**A 61 L 2/10, C 12 N 7/04**

(30) Priority: **14.03.85 US 711780**

(43) Date of publication of application:
**08.10.86 Bulletin 86/41**

(84) Designated Contracting States:
**AT DE FR IT SE**

(71) Applicant: **BAXTER TRAVENOL LABORATORIES, INC.**
**One Baxter Parkway**
**Deerfield, IL 60015(US)**

(72) Inventor: **Dolana, Gary H.**
**24682 Nympha Drive**
**Mission Viejo California 92691(US)**

(74) Representative: **Weinhold, Peter, Dr. et al,**
**Patentanwälte Dipl.-Ing. G. Dannenberg Dr. P. Weinhold**
**Dr. D. Gudel Dipl.-Ing. S. Schubert Dr. P. Barz**
**Siegfriedstrasse 8**
**D-8000 München 40(DE)**

(54) Photodynamic inactivation of virus in therapeutic protein compositions.

㉗ Viruses present in therapeutic protein compositions are inactivated without substantially affecting the activity of the protein by exposing the compositon to light in the presence of a photosensitizer until the viruses are inactivated. The resulting compositions are substantially free of infectious viruses. The method is applicable to the inactivation of hepatitis and retroviruses in blood plasma protein compositions.

Croydon Printing Company Ltd.

This invention relates to a method for the inactivation of viruses in compositions containing therapeutic proteins to produce compositions which are no longer potentially hazardous to patients and medical personnel. More particularly, this invention relates to photodynamic inactivation of viruses in therapeutic blood protein compositions such as factor VIII and IX concentrates, prothrombin complex, and immune globulins.

For the purposes of this invention, the language "inactivation of virus", and "substantially free of infectious virus" as used herein means that the residual titer of biologically infectious virus in products treated according to the method described below is so low that infusion of therapeutic quantities of the product into a plurality of normal animal hosts for the virus in question will not produce statistically significant clinical or serological evidence of infection ($p < 0.1$) in a statistically significant host population. This does not require that the products be sterilized, or completely free of virus, because compositions having a low viral titer may have therapeutic utility where the alternative is a more contaminated material.

Conventional therapeutic methods for treatment of various medical disorders involve administration of blood protein concentrates obtained by fractionation of human blood plasma or by genetic engineering techniques. For instance, Factor VIII or antihemophilic factor is useful against hemophilia; plasminogen is a precursor of plasmin for treatment of acute thromboembolic disorders; immune serum globulin (IgG) is employed in the treatment of congenital gamma globulin deficiency, measles, poliomyelitis and hepatitis A and B; fibronectin has been identified as active in treatment of burns, shock, cancer, etc.; anti-thrombin III is a coagulation inhibitor; cryoprecipitate itself may be used directly for classic hemophilia; Plasma Protein Fraction (human) and albumin are useful in treatment of shock due to burns, crushing injuries, abdominal emergencies and any other cause producing a predominant loss of plasma fluids and not red cells; immune globulin, intravenous (modified immune serum globulin) is a substitute for immune serum globulin administerable in larger quantities; prothrombin complex is useful as a blood-coagulation-promoting preparation for Factor VIII inhibitor patients; a-1-antitrypsin can be employed in the treatment of emphysema; plasma growth hormone corrects pituitary growth deficiency; somatomedin is useful in correcting growth deficiencies; other immune serum globulins, e.g., IgA, IgD, IgE, and IgM, may be employed to treat various immune protein deficiencies; prealbumin is employed to increase immunologic competence; plasminogen-streptokinase complex (U.S. Pat. No. 4,178,368) can be administered to patients for treatment of thromboembolisms; ceruloplasmin, transferrin, haptoglobin, and prekallikrein have reagent and other uses. These blood plasma protein compositions can be prepared commercially by known methods from large pools of individual blood plasma donations. Despite careful screening of individual donors, such plasma pools contain the inherent risk of transmission of undetected viruses.

All plasma units utilized in manufacture of blood protein compositions are tested for the presence of hepatitis B surface antigen using test systems approved for that purpose. These immunological tests, however, are not sufficiently sensitive to detect all potentially infectious units of hepatitis B. In addition, hepatitis virus may be concentrated during the fractionation procedure. Thus, the small amount of undetectable virus that may be contained in a large plasma pool may become significantly infective when it is concentrated several fold.

The development of specific diagnostic tests for hepatitis A and hepatitis B has made it possible to identify a third type of viral hepatitis that is apparently unrelated immunologically to either of the first two. The transmissible agent, non-A, non-B hepatitis, has been confirmed by transmission of the disease to chimpanzees by exposure to pathological material. There are several lines of evidence pointing to more than one non-A, non-B hepatitis virus. These include cases with sequential episodes of apparent acute non-A, non-B hepatitis, variability in epidemiology and clinical syndromes including incubation periods. Presently no verified method exists for detection of non-A, non-B hepatitis viruses. Hepatitis via infusion of blood plasma products, therefore, remains a problem even where the source plasma has been screened for Hepatitis B.

Reports have also linked the occurrence of acquired immunedeficiency syndrome (AIDS) and AIDS-related complex (ARC) with the use of blood and blood plasma products. Studies indicate the disorders are caused by transmission of human T-lymphotropic retrovirus, HTLV-III. A retrovirus, lymphadenopathy virus LAV/IDAV, has also been identified in patients with AIDS and AIDS-related disorders. Both viruses are transmittable through infusion of blood plasma products.

Furthermore, it is conceivable that adventitious viruses other than those responsible for hepatitis and AIDS could contaminate the preparations. Thus a need exists for a process that will inactivate all viruses, including hepatitis and HTLV III, that may be found in these preparations.

Several methods have been used to inactivate infectious viruses in plasma products. For example, hepatitis has been found to be inactivated in both the dry and liquid state by heating to 60°C for 10 hours or more. Aqueous solutions of blood plasma proteins have been heated at this temperature for such time periods; substantial losses in the activities for many of the active blood protein components have been detected. Addition of stabilizers to the solutions have not substantially reduced these losses.

Therapeutic protein products may also be prepared utilizing genetic engineering techniques. DNA encoding for particular therapeutic proteins can be inserted into host cell lines which are manipulated to express the protein in large concentrations. The expressed proteins are equivalent to natural biologic material. The resultant protein products can also contain infectious viruses which may contaminate the host cell lines - especially mammalian cell lines.

Therefore, it is an object of this invention to reduce the risk of transmission of infectious viruses in therapeutic protein products.

A further object of this invention is to provide a method for inactivation of virus in therapeutic protein products without substantially altering the therapeutic proteins.

A further object of this invention is to provide therapeutic protein products which are substantially free of infectious virus.

Further objects appear herein.

I have found that viruses present in compositions containing therapeutic proteins can be inactivated without substantially affecting the activity of the proteins by exposing the composition to light in the presence of a photosensitizer until the viruses are inactivated. The resulting novel compositions contain noninfectious virus and are substantially free of infectious viruses. The preferred compositions are substantially free of infectious hepatitis viruses and retroviruses. The inactivation is monitored by the use of a biological indicator comprising therapeutic protein, photosensitizer and a predetermined viral titer.

Briefly, in the method of this invention, the composition of therapeutic protein is suspended or dissolved in an aqueous medium with an amount of a photosensitizer sufficient to promote photodynamic inactivation of the the viruses in the solution. The photosensitizer-containing composition is then exposed to light for a period of time sufficient to render the composition substantially free of infectious virus.

The products of this invention include compositions comprising therapeutic proteins, photosensitizers and inactivated virus.

"Therapeutic protein" for purposes of this invention includes any biologically active protein which has qualities which make it useful in the treatment of medical disorders. Examples of such proteins include human blood plasma proteins such as factor VIII, Von Willebrand factor, factor IX, factor X, factor XI, Hageman factor, the activated forms of such factors, prothrombin, anti-thrombin III, fibronectin, plasminogen, immune serum globulin, modified immune globulin, albumin,  -1-antitrypsin, and prekallikrein.

"Therapeutic protein composition" for purposes of this invention includes any composition which comprises a therapeutic protein. The term also includes proteins which are employed for diagnostic purposes. The therapeutic protein can, but need not be, present in therapeutically effective amounts. Purification and concentration of the protein can occur after inactivation of the virus.

Therapeutic protein compositions with which this invention is useful are any compositions which run the risk of viral contamination. Such therapeutic protein compositions would include compositions obtained from a population which has a risk of viral contamination or compositions which have been exposed to potential infection through subsequent handling. Examples include blood plasma protein compositions obtained from human donors and genetically-engineered protein compositions obtained from mammalian host cell lines.

"Photosensitizer" as defined for purposes of this invention is any compound which absorbs light of one or more defined wavelength and subsequently transfers the absorbed energy to an energy acceptor. Photosensitizers which are useful in this invention include such compounds which preferentially adsorb to nucleic acids, thus focussing photodynamic effect upon microorganisms and viruses with little or no effect upon accompanying proteins. Such photosensitizers will be obvious to the ordinary artisan without undue experimentation. Examples of such photosensitizers include, but are not limited to, porphyrins, psoralens, dyes such as neutral red, methylene blue, acridine, toluidines, flavine and phenothiazine derivatives. Preferred photosensitizers are compounds which are not toxic and do not present a biohazard, therefore eliminating the need for further purification or removal steps. These include compounds which are naturally occuring within the body such as protoporphyrin, or compounds which have been approved for other therapeutic uses at much higher concentrations, such as chlorpromazine.

The choice of photosensitizer will be dependent upon the therapeutic protein composition used. The components of the therapeutic protein composition can render some photosensitizers ineffective or can enhance the activity of other photosensitizers. For instance, the photodynamic actively of protoporphyrin is ineffective in inactivating viruses in the presence of albumin. Therefore, protoporphyrin can not be used to inactivate viruses in compositions which inherently contain albumin. It would, however, be possible to use protoporphyrin in an albumin-free composition; albumin advantageously can be added later to stop photodynamic activity.

Addition of the photosensitizer and photodynamic inactivation of the virus can occur at any point along the processing of the therapeutic protein and will be dependent upon several factors such as the therapeutic protein composition, the photosensitizer, processing steps, processing time, processing conditions, etc. For example, with blood plasma protein compostions, photosensitizers such as protoporphyrin which are natural products can be added just prior to sterile filtration. When using chlorpromazine for the same products, it is desirable to remove as much of the photosensitizer as possible. Therefore, it may be preferable to add the photosensitizer prior to ultrafiltration or any other step which would result in removal of the compound from the product.

Inactivation conditions will vary dependent upon the photosensitizer and the therapeutic protein composition employed. The therapeutic protein composition is treated in a liquid form. The therapeutic protein can be dried and then reconstituted for treatment. Concentration of the composition generally has no effect on the invention. Concentration of the photosensitizer will vary dependent upon the photosensitizer and the composition. Optimal concentrations can be determined by the artisan without undue experimentation. Temperature of the composition generally will have no effect on inactivation. The pH of the solution, although it may have some effect on particular photosensitizers, is generally not significant. In blood plasma protein compositions, the pH will generally range from about 6.0 to about 8.0. Many photosensitizers, such as protoporphyrin, will require that the reaction be carried out in the presence of molecular oxygen.

The wavelength of the light used for inactivation will vary dependent upon the photosensitizer used. The wavelength is chosen to be one at which the phosensitizer absorbs, but also one at which the least damage is done to the protein. It generally will be necessary to filter out undesirable wavelengths. In a preferred model, where chlorpromazine is used to inactivate virus in an antihemophilic factor (AHF) composition, the photosensitizer absorbs at both 254 nm and 306.4 nm. The light of the lower wavelengths is filtered out because light at those wavelengths inactivates AHF as well as the virus.

The materials through which the composition is exposed to light will be dependent upon the wavelength of absorption of the photosensitizer. Glass will generally absorb wavelengths in the ultraviolet range, making it an inappropriate medium for some of the photosensitizers. Preferred materials would include polymers which do not absorb at the desired absorption peak but which filter out undesirable wavelengths.

The length of time of exposure to ensure substantial inactivation of the virus will again be dependent upon the photosensitizer employed. For instance, substantial inactivation of virus may occur after approximately five minutes when using protoporphyrin; similar levels of inactivation may require ten to twenty minutes when utilizing chlorpromazine.

While a standardized time period can be used for uniform lots of therapeutic protein compositions, it is preferable to assay the composition for viral biological activity to determine whether or not the inactivation is complete. The virus to be assayed may be the one which is suspected to contaminate the composition, e.g., hepatitis B, non-A, non-B hepatitis, HTLV III, or the virus may be one which has been preselected for use in a biological indicator. A biological indicator acts as a product surrogate in that the inanimate portions are designed to mimic the product while a living organism is selected to mimic the expected contaminants.

0196515

-10-

Biological indicators heretofor have been articles containing sterilant resistant cellular organisms or spores of such organisms. These known biological indicators are most commonly available as dry, bibulous strips impregnated with the heat resistant spores of certain bacteria, e.g., bacillus, which in turn are encased in packages which are permeable to the sterilizing agent, e.g., steam. The biological indicator herein provided is a composition comprising therapeutic protein, a photosensitizer and a predetermined titer of an infectious virus which is not a candidate for infection in the particular therapeutic protein composition.

The therapeutic protein mimics the effect of the protein in the product which is to undergo the virus inactivation process. Thus the indicator protein may be the same or different from the proteins in the product to be treated, either in terms of identity, proportion or species of origin. Thus a variety of commonly available proteins may be employed, e.g. immune globulin, albumin, antihemophilic factor, antithrombin III, clotting enzymes and mixtures thereof. In a preferred embodiment the indicator protein composition is substantially the same as that in the product to be treated. In fact, suitable indicators may be prepared from aliquots of each protein lot to be treated.

The virus is preferably not normally infectious for humans, thus reducing containment costs. For the safety of laboratory workers and to ensure integrity of treated product it is important that the virus not be a candidate or known agent responsible for infection upon infusion into humans. Such viruses are hazardous and their introduction into the therapeutic protein manufacturing environment should be avoided. The concentration of virus will range about from 3 to 8 $\log_{10}$ plaque-forming units in about from 0.01 to 1 gram of protein.

Indicators are used by exposing them to the same conditions as are used to inactivate the suspected virus in the composition to be treated and then assaying for the virus, if any, which remains active in the biological indicator. It is preferred to place biological indicators in among the product vials and then to treat; this helps ensure that the inactivation conditions are substantially identical.

Suitable viruses for use in the biological indicators include bacterial, plant and animal viruses. The animal viruses are preferably not normally infectious for humans for safety reasons. Also, the virus ideally should not be infectious for the donor species of the protein used in the biological indicator. The reason for this is that such protein could be contaminated with antibody to the virus as a result of prior infection or vaccination of the donor, and the antibody may interfere with viral inactivation studies. Certain human viruses can be employed under carefully defined conditions.

Exemplary viruses include picornaviruses such as encephalomyocarditis virus (EMC) mouse encephalomyelitis virus, simian enteroviruses and retroviruses; togaviruses including sindbis, semliki forest virus, western equine encephalitis virus and yellow fever virus; retroviruses such as rous sarcoma virus; paramyxoviruses such as newcastle disease virus; papoviruses including polyoma virus and simial virus 40; herpes viruses such as herpes simplex virus (HSV), pseudorabies virus and infectious Govine rhinotrachitis; members of the adenovirus family such as infectious canine hepatitis and adeno virus; members of the rhobdovirus group such as vesicular stoniatitis virus (VSV); and bacteriophage such as R17, lambda, T1, T2, T4, T7 and Phi X 174. Mixtures of the viruses also may be used.

Suitable methods for determining the amount of infectious viruses in the treated product or in biological indicators are quite conventional and will be apparent to the artisan. One such method is known as the plaque-forming test. In this method, dilutions of test sample in a physiological diluent are contacted with an immobilized layer of living, susceptible cells; time is allowed for the viruses to adsorb to the cells; the cells are overlaid with a growth medium gel; and other conditions are set to otherwise optimize survival of the cell culture. Viruses that infect and multiply within the cells are localized by the agar overlay. Virus spread is from the primary infected cells to adjacent cells producing a circular area of cellular degeneration or foci called a plaque. These foci are usually visualized by staining the monolayer with a vital stain, neutral red. The foci of infected cells fail to take up the vital stain and thus appear as clear areas against a colored background. Accordingly, the number of plaques is a measure of the infectivity of the test sample.

Other assays that may be employed include in vivo tests for infectivity towards susceptible hosts. Such methods are well known. Hepatitis B must be assayed in this manner because it has not been possible heretofore to culture the virus in vitro. The usual procedure is to inoculate hepatitis-B antibody negative chimpanzees or marmosets with the samples to be evaluated, and then observe animals for clinical, biochemical and serological evidence of hepatitis-B infection.

Immune assays for normal viral antigens in treated samples will yield reasonably conclusive results only if no antigen is detectable and, as noted above, the immune assay is sufficiently sensitive. If both requirements are met, the treatment has been sufficiently rigorous to completely denature the viral eptitopic sites. It is generally safe to assume that such treatment also has rendered the viruses biologically inactive, and thus noninfectious.

However, determining the point at which viral inactivation has occurred by the use of such assays is not preferred because the conditions needed to denature antigens will be far more severe in most cases that those necessary to inactivate the virus infectivity. Thus unnecessary losses in product will occur before the sample can be verified as noninfectious.

The following examples are intended to illustrate more fully the nature of the present inventions without acting as a limitation upon its scope.

EXAMPLES

In the following examples, a known concentration of a photosensitizer was added to protein material, viral material, or a combination of the two. The mixture was then exposed to light of a defined wavelength for a specified period of time.

Two photosensitizers were used. Protoporphyrin was selected because it is a natural breakdown product of heme and thus may be present in all plasma derived products. Chlorpromazine, 10-(3-dimethyl-aminopropyl)-2-chlorphenothiazine, is sold under the brand name Thorazine and is used as a sedative.

Protein compositions utilized include cell culture growth medium, gelatin, antihemophilic factor (AHF) manufactured by Hyland Therapeutics division of Travenol Laboratories, Inc. and factor IX complex (FIXC) also manufactured by Travenol.

Several light sources were used. A U.V. light source derived from a high pressure mercury vapor quartz lamp (85 watts) was employed. This source provided a wide band of light in the 390 to 410 nm range. A filter was used to remove the radiation below 350 nm in wave length. Alternatively a super diazo blue fluorescent lamp was used which has a peak emission near the absorption peak of the porphyrins employed.

-14-

Virus infectivity of the samples was measured using the technique of R. Dulbecco, "Production of plaques in monolayer issue culture by single particles of an animal virus," Proc Nat'l Acad Sci U.S., 38:747-52(1952). The sample to be assayed was serially diluted in serum free MEM or phosphate buffered salt solution. Two tenths ml of each dilution was added to a cell monolayer and incubated for an hour. The monolayer was then overlaid with a medium containing agarose. After an incubation time, which varied from to two to fourteen days depending upon the virus, the placques were counted with the addition of neutral red solution.

Factor VIII C and Factor IX activity was determined by the one-stage activated partial thromboplastin time assay using Kaolin as an activator and human Factor VIII or Factor IX deficient plasma as a substrate. The activated partial thromboplastin time assay, better known as APTT, is a screening test for deficiencies in the intrinsic system of coagulation of which both Factor VIII and Factor IX are a part. It involves the addition of the test sample to Factor VIII or Factor IX deficient plasma and measuring the degree of correction of the clotting time. This is then compared to a curve constructed using various dilutions of a lyophilized concentrate (reference) of known potency. The result is expressed in terms of percentage of the reference. Both sample and refernce are prediluted in Factor VIII deficient substrate plasma to an approximate activity of 1 U/mL. From the percentage of the reference, the potency can be estimated.

EXAMPLE I

The following example demonstrates the ability of the photodynamic inactivation process to inactivate a biological marker virus suspended in protein concentrate and the importance of employing the proper concentration of photosensitizer. Protoporphyrin IX was employed as the photosensitizer. The light source, its distance from the product-photosensitizer-virus mixture, and the protein concentration were held constant. The control was virus suspended in cell culture media without addition of serum of bovine origin.

Light Source: Mercury Arc Lamp w/Filter
Photosensitizer: Protoporphyrin

| Virus | Product | Photo-sensitizer Concen-tration | Amount of Virus Inactivity [Pfy/mL] |
|---|---|---|---|
| Sindbis | AHF | $1.6 \times 10^{-4}$ | 5.2 $\log_{10}$ in 120 sec. |
| Sindbis | AHF | $1.7 \times 10^{-4}$ M | 6.5 $\log_{10}$ in 120 sec. |
| Sindbis | AHF | $1 \times 10^{-6}$ M | 6.0 $\log_{10}$ in 60 sec. |
| Sindbis | AHF | $5 \times 10^{-6}$ M | 6.0 $\log_{10}$ in 30 sec. |
| Sindbis | FIXC | $5 \times 10^{-6}$ M | 6.0 $\log_{10}$ in 120 sec. |
| Sindbis | FIXC | $1 \times 10^{-5}$ M | 6.0 $\log_{10}$ in 60 sec. |

EXAMPLE II

The data below demonstrates that the combination of photosensitizer (protoporphyrin) and light which inactivate a marker virus within three minutes of light exposure does not significantly reduce the Factor VIII activity in the liquid state when exposed for 5 minutes under identical conditions. It can also be seen that higher concentrations of photosensitizer does result in considerable loss of activity.

Light Source: Mercury Arc Lamp w/Filter
Product: AHF
Photosensitizer: Protoporphyrin

| Photosensitizer Concentration | Exposure Time(sec) | Average Units/ml | % Change (Ref: Bulk) |
|---|---|---|---|
| $1 \times 10^{-6}$M | Bulk | 30.3 | -- |
| | 0 | 34.7 | +15% |
| | 60 | 29.8 | - 2 |
| | 120 | 29.9 | - 1 |
| | 300 | 29.0 | - 4 |
| $1 \times 10^{-6}$M | Bulk | 35.4 | -- |
| | 0 | 33.4 | - 6 |
| | 60 | 35.3 | 0 |
| | 120 | 32.3 | - 9 |
| | 300 | 36.0 | + 3 |
| $1 \times 10^{-6}$M | Bulk | 34.0 | -- |
| | 0 | 30.0 | -12 |
| | 60 | 30.4 | -11 |
| | 120 | 30.2 | -11 |
| | 300 | 31.1 | - 9 |
| $1 \times 10^{-6}$M | Bulk | 30.4 | -- |
| | 0 | 29.5 | - 3 |
| | 60 | 29.2 | - 4 |
| | 120 | 28.2 | - 7 |
| | 300 | 26.8 | -12 |
| $5 \times 10^{-6}$M | Bulk | 33.1 | -- |
| | 0 | 42.4 | +28 |
| | 60 | 37.1 | +12 |
| | 120 | 34.4 | + 4 |
| | 300 | 32.1 | - 3 |

| Photosensitizer Concentration | Exposure Time(sec) | Average Units/ml | % Change (Ref: Bulk) |
|---|---|---|---|
| 5 X 10^-6 M | Bulk | 34.0 | -- |
| | 0 | 33.2 | - 2 |
| | 60 | 29.1 | -14 |
| | 120 | 29.5 | -13 |
| | 300 | 32.6 | - 4 |

EXAMPLE III

The data below indicates results similar to Example II with respect to Factor IX complex.

Light Source: Mercury Arc Lamp w/Filter
Product: FIXC
Photosensitizer: Protoporphyrin

| Photosensitizer Concentration | Exposure Time(sec) | Average Units/ml | % Change (Ref: Bulk) |
|---|---|---|---|
| 1 X 10^-6 M | Bulk | 20.5 | -- |
| | 0 | 24.6 | +20 |
| | 60 | 21.0 | + 2 |
| | 120 | 19.9 | - 3 |
| | 300 | 20.5 | 0 |
| 1 X 10^-6 M | Bulk | 19.6 | -- |
| | 0 | 19.3 | - 2 |
| | 60 | 18.6 | - 5 |
| | 120 | 19.9 | + 2 |
| | 300 | 17.2 | -12 |
| 5 X 10^-6 M | Bulk | 20.5 | -- |
| | 0 | 21.2 | + 3 |
| | 60 | 15.7 | -23 |
| | 120 | 15.6 | -24 |
| | 300 | 18.1 | -12 |
| 5 X 10^-6 M | Bulk | 22.8 | -- |
| | 0 | 26.6 | +17 |
| | 60 | 19.0 | -17 |
| | 120 | 23.0 | + 1 |
| | 300 | 19.1 | -16 |

| Photosensitizer Concentration | Exposure Time(sec) | Average Units/ml | % Change (Ref: Bulk) |
|---|---|---|---|
| $1 \times 10^{-5}M$ | Bulk | 22.2 | -- |
|  | 0 | 19.2 | -14 |
|  | 60 | 15.1 | -31 |
|  | 120 | 11.8 | -47 |
|  | 300 | 7.9 | -65 |

## EXAMPLE IV

This example demonstrates the utility of chlorpromazine as a photosensitizer for photodynamic inactivation in AHF products. By employing chlorpromazine at a concentration of $1 \times 10^{-4}M$, more than 5 logs of each of the three marker viruses were inactivated when exposed to light for less than ten minutes.

Light: Mercury Arc Lamp
Product: AHF
Photosensitizer: Chlorpromazine $(1 \times 10^{-5}M)$

| Exposure Time (minutes) | VIRUS | | | | | |
|---|---|---|---|---|---|---|
| | Sindbis | | VSV* | | HSV** | |
| | Virus Pool | Product | Virus Pool | Product | Virus Pool | Product |
| 0 | 5.9[1] | 5.1 | 7.4 | 6.5 | 4.9 | 4.8 |
| 10 | 0.0 | 0.0 | 3.2 | 4.5 | 2.9 | 0.0 |

[1] Plaque-forming units per mL
*Vesicular stomatitis virus
**Herpes Simplex virus

Light:  Mercury Arc Lamp
Product:  AHF
Photosensitizer:  Chlorpromazine $(1 \times 10^{-4} M)$

| Exposure Time | VIRUS | | | | | |
| | Sindbis | | VSV* | | HSV** | |
| | Virus Pool | Product | Virus Pool | Product | Virus Pool | Product |
|---|---|---|---|---|---|---|
| 0 | 5.9[1] | 5.1 | 7.8 | 7.3 | 6.5 | 5.5 |
| 10 | 0.0 | 0.0 | 0.0 | 0.0 | 0.0 | 0.0 |

[1] Plaque-forming units per mL.
*Vesicular stomatitis virus
**Herpes Simplex virus

Further it can be seen that some inactivation of Factor VIII activity does occur.  The data below indicates that the loss in activity may be the result of the light source and not photodynamic action.

Light:  Mercury Arc Lamp
Product:  AHF
Photosensitizer:  Chlorpromazine  $(1 \times 10^{-4} M)$

| Sample Description | Units/ml | % Change* (Ref: Bulk) |
|---|---|---|
| Bulk | 28.2 | |
| Sensitizer added-no light | 27.3 | - 7% |
| Sensitizer added-10 min light | 20.0 | -29% |

*Percent change in Factor VIII coagulant activity.

Light: Mercury Arc Lamp
Product: AHF
Photosensitizer: Chlorpromazine ($1 \times 10^{-5}$M)

| Sample Description | Units/ml | % Change* (Ref: Bulk) |
|---|---|---|
| Bulk | 32.4 | |
| Sensitizer added-10 min light | 24.8 | -23 |
| Sensitizer added-20 min light | 18.2 | -43 |
| No sensitizer added-20 min light | 19.2 | -41 |
| Sensitizer added-no light | 30.1 | - 7 |

*Percent change in Factor VIII coagulant activity.

EXAMPLE V

The following data supports photodynamic inactivation of LAV using indirect methods. AHF was seeded with LAV virus. In the first set of data, Protoporphyrin was added at a concentration of 5 X $10^{-6}$M. The mixture was exposed to light ffrom a 65 watt Super Diazo Blue lamp at a distance of 10 cm. Albumin was added to the samples after inactivation to inhibit any inactivation from extraneous light. The product was lyophilized and subjected to a reverse transcriptase assay. Results show almost 100% reduction in LAV reverse transcriptase activity between 2 to 5 minutes of exposure.

In the second set of data, LAV was seeded into an AHF composition. Chlorpromazine (1 X $10^{-4}$M) was added as a photosensitize and the mixture was exposed to unfiltered light from an 85 watt high pressure mercury arc lamp at a distance of 10 cm. The product was lyophilized and analyzed for reverse transcriptase activity. Results are reported in counts per minute.

Light Source:  Super Diazo Blue
Product:  AHF
Photosensitizer:  Protoporphyrin (5 X $1^{-6}$M)

| Exposure time (minutes) | C.P.M. |
|---|---|
| Positive Control | 14,096 |
| 0 | 4,686 |
| 1 | 4,766 |
| 2 | 2,054 |
| 5 | 1,452 |
| Background | 1,022 |

Light Source:  Mercury Arc Lamp
Product:  AHF
Photosensitizer:  Chlorpromazine (1 X $10^{-4}$M)

| Exposure time (minutes) | C.P.M. |
|---|---|
| Positive Control | 34,208 |
| 0 | 36,192 |
| 5 | 27,100 |
| 10 | 6,814 |
| 20 | 4,742 |
| Background | 1,022 |

CLAIMS

1. A method for inactivating virus in a therapeutic protein composition suspected to contain such virus, characterized in that it comprises:

      a) adding a photosensitizer to the composition; and

      b) exposing the composition to light of a defined wavelength until the composition is substantially free of said virus in the infectious form.

2. The method of claim 1 wherein the therapeutic protein composition comprises a blood plasma protein, e.g. selected from the group consisting of factor VIII, Von Willebrand factor, factor VII, factor IX, factor X, factor XI, Hageman factor, activated factor VII, activated factor IX, activated factor X, activated factor XI, activated Hageman factor, prothrombin, anti-thrombin III, fibronectin, plasminogen, immune serum globulin, modified immune serum globulin, albumin, -1-antitrypsin, prekallikrein and mixtures thereof.

3. The method of one or both of the claims 1 to 2, wherein the photosensitizer is selected from the group consisting of porphyrins, psoralens, neutral red, methylene blue, acridines, toluidines, flavine, and phenothiazine derivatives.

4. The method of claim 3 wherein the photosensitizer is protoporphyrin, the composition being substantially free of albumin.

5. The method of one or more of the claims 1 to 4, wherein the composition is exposed to light in the presence of molecular oxygen.

6. The method of one or more of the claims 1 to 3 and 5 wherein the photosensitizer is chlorpromazine.

7. The method of one or more of the claims 1 to 6 wherein the composition is a biological indicator.

8. The method of claim 7, wherein the virus is selected from the group consisting of sindbis virus, herpes simplex, vesicular stoniatitis,a bacteriophage or encephalomyocarditis virus.

9. A composition containing blood plasma protein, photosensitizer and noninfectious virus, said virus having been made noninfectious by a method comprising exposing the virus to light in the presence of the photosensitizer until the virus is inactivated, which composition is substantially free of said virus in the infectious form.

10. The composition of claim 9, wherein the therapeutic protein is a blood plasma protein, e.g. selected from the group consisting of factor VIII,Von Willebrand factor, factor VII, factor IX, factor X, factor XI, Hageman factor, activated factor VII, activated factor IX, activated factor X, activated factor XI, activated Hageman factor, prothrombin, anti-thrombin III, fibronectin, plasminogen, immune serum globulin, modified immune serum globulin, albumin, -1-antitrypsin, prekallikrein and mixtures thereof.

11. The composition of one or both of the claims 9 to 10 wherein the photosensitizer is selected from the group consisting of porphyrins, psoralens, neutral red, methylene blue, acridines, toluidines, flavine and phenothiazine derivatives.

12. The composition of claim 11 wherein the photosensitizer is protoporphyrin,the composition being substantially free of albumin.

13. The composition of one or more of the claims 9 to 12, wherein the virus is exposed to light in the presence of molecular oxygen.

14. The composition of one or more of the claims 9 to 11 and 13 wherein the photosensitizer is chlorpromazine.

15. The composition of one or more of the claims 9 to 14 wherein the composition is a biological indicator.

16. The composition of claim 15 wherein the virus is selected from the group consisting of sindbis virus, herpes simplex, vesicular stoniatitis virus, a bacteriophage or encephalomyocarditis virus.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP-A-0 124 363 (ADVANCED GENETICS RESEARCH INSTITUTE) * Page 3, lines 16-30; claims 1-12; page 4, lines 26-32 * | 1-3,8-11 | A 61 L 2/08<br>A 61 L 2/10<br>C 12 N 7/04 |
| Y | | 4,5,12,13 | |
| Y | CHEMICAL ABSTRACTS, vol. 91, no. 19, 5th November 1979, page 235, abstract no. 153200u, Columbus, Ohio, US; J.D. SPIKES et al.: "Energy transfer mechanisms in photobiological reactions", & ENERGY RES. ABSTR. 1979, 4(12), Abstr. No. 34905 | 4,12 | |
| Y | US-A-4 402 318 (M.R. SWARTZ) * Columns 3,4; claims 1-6 * | 5,13 | TECHNICAL FIELDS SEARCHED (Int Cl 4)<br><br>A 61 L<br>C 12 N |

The present search report has been drawn up for all claims

| Place of search | Date of completion of the search | Examiner |
|---|---|---|
| THE HAGUE | 24-06-1986 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82